# EUROPEAN PATENT APPLICATION

(11) **EP 4 005 504 A1**
(43) Date of publication of application: **01.06.2022**
(21) Application number: 21207559.2
(22) Date of filing: 10.11.2021
(51) Int. Cl.: A61B 17/072

(54) **SURGICAL CLIP APPLIER**

(30) Priority: 30.11.2020 KR 20200164398
(71) Applicant: Hwainmedi Co., Ltd., Chungcheongnam-do 31094 (KR)
(72) Inventor: LEE, Seoung Won, Cheonan-si, Chungcheongnam-do (KR)
(74) Representative: BCKIP

(57) **Abstract**

The present disclosure relates to a surgical clip applier capable of automatically loading and moving a titanium or polymer clip to an application position by using pneumatic pressure and operating a handle unit only once. The present disclosure also relates to a surgical clip applier having a ligation unit that ligates a titanium or polymer clip in a loaded state, thereby improving stability and accuracy in applying the clip and improving convenience for patients and users. The present disclosure also relates to a surgical clip applier, in which a handle unit may be used in common for titanium and polymer clips, only a shaft unit may be replaced to be selectively and detachably installed at one side of the handle unit depending on the types of titanium and polymer clips, and only a pneumatic tank may be conveniently and quickly replaced when pneumatic pressure in the pneumatic tank is exhausted, thereby reducing surgery costs and preventing waste of resources.

## Description

### [FIELD OF THE DISCLOSURE]

The present disclosure relates to a surgical clip applier, and more particularly, to a surgical clip applier capable of automatically loading and moving a titanium or polymer clip to an application position by using pneumatic pressure and operating a handle unit only once. The present disclosure also relates to a surgical clip applier having a ligation unit that ligates a titanium or polymer clip in a loaded state, thereby improving stability and accuracy in applying the clip and improving convenience for patients and users. The present disclosure also relates to a surgical clip applier, in which a handle unit may be used in common for titanium and polymer clips, only a shaft unit may be replaced to be selectively and detachably installed at one side of the handle unit depending on the types of titanium and polymer clips, and only a pneumatic tank may be conveniently and quickly replaced when pneumatic pressure in the pneumatic tank is exhausted, thereby reducing surgery costs and preventing waste of resources.

### [BACKGROUND OF THE DISCLOSURE]

In general, during a surgical procedure, a practitioner should close or ligate blood vessels and other conduits at a surgical site prior to incision in order to prevent excessive bleeding in the patient and reduce the risk of other complications caused by infection.

In addition, during a surgical procedure such as resection of an organ, a procedure of cutting a vein or artery or a procedure of hemostasis is frequently performed. In particular, in order to prevent excessive bleeding during a procedure of cutting the blood vessel, an operation of stopping bleeding is performed on both sides of the blood vessel to be cut before cutting the blood vessel.

Typically, hemostasis of blood vessels is performed by suturing or ligating the blood vessels using surgical threads. However, in order to perform the hemostasis of the blood vessel using the surgical thread as described above, it is necessary to manually suture or ligate the blood vessel using needles and suture materials, which causes a problem in that a large amount of time is required to perform the surgical procedure such as endoscopic surgery which is limited in space and time, and it is difficult to perform the surgical procedure.

Meanwhile, there is always the likelihood of damage to blood vessels during the vascular ligation using the surgical thread or the likelihood of detachment due to loosening or slipping of the ligated surgical thread. In particular, this likelihood is increased in a larger blood vessel and may cause serious problems of massive bleeding after surgery.

As described above, recently, in order to solve the problem caused by using the surgical thread as described above, a surgical clip has been proposed and widely used. The above-mentioned surgical clip may be applied relatively easily and quickly in comparison with the method using the surgical thread, so the use of the surgical clip is increasing not only in open type surgery but also in endoscopic surgery, and a surgical clip applier for applying the clip is also being developed in various ways.

Therefore, recently, ligation methods using the clips made of various materials are widely used, and various surgical clips for this purpose have been proposed. In addition, the types of surgical clips are classified into a titanium clip and a polymer clip in accordance with the purposes.

However, in the case of the surgical clip applier in the related art, a trigger needs to be finely and precisely adjusted to load the clip to an application position and sequentially apply the clips one by one. When the surgical clip applier is operated by a practitioner with low skill or the clip is loaded and applied by a mechanical mechanism, a defect may occur or the clip is not smoothly applied or loaded because of the manipulation by the mechanical mechanism and the configuration including a plurality of devices, such that several clips are applied at once, which causes a problem in that a device defect occurs, stability and accuracy deteriorate, and reliability is decreased. In severe cases, there is a problem in that the time required to perform a surgical operation on a patient increases and the recovery is delayed.

In addition, because the surgical clip applier in the related art cannot automatically load the clip to the application position, reliability is decreased, accuracy of an operation deteriorates, and operating time is increased, which causes a problem in that a patient's recovery is decreased, a practitioner's fatigue is increased, and the patient's life is threatened because of an increase in medical accidents.

Moreover, in the case of the surgical clip applier in the related art, only the surgical clip applier dedicated to the application of the titanium clip is used to apply the titanium clip, and only the surgical clip applier dedicated to the application of the polymer clip is used to apply the polymer clip. That is, because the titanium clip and the polymer clip have a large difference in their characteristics and advantages and disadvantages, the mechanisms and configurations for applying the titanium clip and the polymer clip are different from each other. Because different configurations need to be provided, that is, because the surgical clip applier configured to apply the titanium clip applies only to the titanium clip, and the surgical clip applier configured to apply the polymer clip applies only to the polymer clip. For this reason, a practitioner needs to separately and independently purchase or use the independent surgical clip appliers to use the titanium clip and the polymer clip, which causes problems in that operation costs are increased, a patient's economic burdens are increased, inconvenience is caused to the practitioner, and waste of resources occurs.

In addition, in the case of the surgical clip applier in the related art, because the respective components or configurations such as a shaft and a handle unit are not optimized, assembled, and produced in modularized forms, manufacturing costs and manufacturing time are increased, and miniaturization cannot be achieved. For this reason, there is a problem in that the equipment becomes large, which causes inconvenience to a user or operator, increases the storage and transport costs, ultimately increases the burden of the patient's operating expenses, increases sale price, increases medical insurance premiums, and causes waste of national finances.

### [Documents of Related Art]

### [Patent Documents]

(Patent Document 1) Korean Patent Laid-Open No. 10-2012-0018788
(Patent Document 2) Korean Patent Laid-Open No. 10-2012-0028911
(Patent Document 3) Korean Patent Laid-Open No. 10-2015-0112760
(Patent Document 4) Korean Patent Laid-Open No. 10-2020-0073008

### [Disclosure]

### [SUMMARY]

The present disclosure has been made in an effort to solve the above-mentioned problems of the present disclosure, and an object of the present disclosure is to provide a surgical clip applier capable of automatically loading and moving a titanium or polymer clip to an application position by using pneumatic pressure and operating a handle unit only once. Another object of the present disclosure is to provide a surgical clip applier having a ligation unit that ligates a titanium or polymer clip in a loaded state, thereby improving stability and accuracy in applying the clip regardless of the types of clips, maximizing reliability, improving convenience for users, shortening surgical operation time, and implementing quick recovery of a patient. Still another object of the present disclosure is to provide a surgical clip applier, in which a handle unit may be used in common for titanium and polymer clips regardless of the types of clips, only a shaft unit detachably coupled and installed to one side of the handle unit so as to be selectively replaceable may be replaced depending on the types of clips including the titanium and polymer clips to be applied by the shaft unit and a ligation unit, only a cartridge unit, the clip, or a pneumatic tank may be conveniently and quickly replaced when all the clips are consumed or pneumatic pressure is exhausted, and the handle unit may be repeatedly used, thereby reducing surgery costs and preventing waste of resources. Yet another object of the present disclosure is to provide a surgical clip applier capable of improving assembly properties and productivity, reducing manufacturing costs and manufacturing time by improving assembly properties and productivity, achieving convenience for storage and transport by making the surgical clip applier compact, and thus maximizing a consumer's satisfaction by reducing manufacturing costs.

An exemplary embodiment of the present disclosure provides a surgical clip applier, which applies a titanium or polymer clip for ligating a blood vessel or tubular tissue, the surgical clip applier including: a handle unit configured to load the titanium or polymer clip by pneumatic pressure and ligate the blood vessel or tubular tissue with the titanium or polymer clip; a shaft unit selectively and detachably installed at one side of the handle unit depending on the titanium or polymer clip loaded by the pneumatic pressure; and a ligation unit disposed at one side of the shaft unit and configured to ligate the clip, in which the handle unit includes: a housing part having a horizontal portion extending in a horizontal direction and a grip part disposed below the horizontal portion and extending in a vertical direction; an adjustment part detachably coupled and installed at one side of the horizontal portion and configured to allow a part of the shaft unit to be inserted thereinto; a pneumatic pressure supply part tiltably installed on the grip part and configured to supply the pneumatic pressure for loading the titanium or polymer clip; a lever part having a trigger part turnably installed on the grip part and configured to load and ligate the titanium or polymer clip depending on a turning operation of the trigger part; and an operation part installed in the horizontal portion and configured to operate in conjunction with the lever part.

In the surgical clip applier according to the present disclosure, the adjustment part of the handle unit may include: a coupling part disposed at a tip of the grip part; and a hollow portion configured to penetrate the inside of the coupling part, and the pneumatic pressure supply part may include: a cover part coupled to the grip part so as to be tiltable with respect to a tilting shaft disposed at the other end of the grip part; a turning part coupled to the cover part so as to be turnable with respect to a turning shaft disposed on a part of the cover part and configured to turn in conjunction with the cover part; a storage part coupled to the turning part so as to be rotatable with respect to a rotary shaft disposed at one end of the turning part and configured to rotate in conjunction with the turning part; and a pneumatic tank separably inserted into the storage part.

In the surgical clip applier according to the present disclosure, the lever part of the handle unit may include: a drive part installed in the grip part and disposed adjacent to the trigger part so as to turn in a direction opposite to the turning direction of the trigger part; an actuator part installed in the grip part and disposed adjacent to the drive part so as to be turned in a direction opposite to the turning direction of the drive part; a transmission part installed in the horizontal portion in a state in which the other side thereof is fixed and coupled to a part of the horizontal portion so that one side thereof moves upward or downward depending on the turning direction of the actuator part; and a fixing part installed in the horizontal portion and disposed adjacent to the transmission part in a state in which the other side thereof is fixed and coupled to a part of the horizontal portion so that one side thereof moves upward or downward in conjunction with the transmission part.

In the surgical clip applier according to the present disclosure, the trigger part of the lever part of the handle unit may include a concave groove portion recessed at an upper end of the trigger part, the drive part may include: a main body part having a driving shaft disposed at a center thereof and configured to turn with respect to the driving shaft in the direction opposite to the turning direction of the trigger part; a first leg part extending in the horizontal direction from an upper portion of the main body part; a second leg part extending in the vertical direction from a lower portion of the main body part; and a ring part extending vertically downward from a tip of the first leg part, the actuator part may include: a body part having a rotation shaft disposed on a part thereof and configured to turn with respect to the rotation shaft in the direction opposite to the turning direction of the drive part; a catching projection part recessed vertically downward from an upper portion of the body part so as to be selectively fixed and coupled to the ring part in the turning direction of the actuator part; and a guide part protruding adjacent to the transmission part so as to have a predetermined curvature at a vertical upper end of the body part, the transmission part may include: a body part having one side formed as a free end, and the other side fixed to a part of the horizontal portion and extending in the horizontal direction; a cam follower part formed at one side of the body part so as to have a curvature corresponding to a curvature of the guide part and configured to slide along a guide surface of the guide part; and a pressing part protruding from a center of a lateral surface of the body part toward the fixing part, and the fixing part may include: a support part having one side formed as a free end, the other side fixed to a part of the horizontal portion and extending in the horizontal direction, the support part being parallel to the body part and disposed adjacent to the body part; a stopper part extending vertically upward from one side tip of the support part; and a stepped part formed in a stepped manner by penetrating a center of the support part so as to correspond to the pressing part so that the support part moves upward or downward in conjunction with the upward or downward movement of the body part, the stepped part being in close contact with the pressing part.

In the surgical clip applier according to the present disclosure, the operation part of the handle unit may include: a base part fixed and installed at the other side in the horizontal portion and having a joint part disposed in the base part and coupled to the pneumatic tank so as to communicate with the pneumatic tank by reciprocatingly and rectilinearly moving; a loading part provided at the other side in the horizontal portion, disposed adjacent to the base part, and configured to load the titanium or polymer clip depending on the upward or downward movement of the stopper part; a flow path part having one side communicating with the loading part, and the other side communicating with the base part, and configured to transmit the pneumatic pressure, discharged from the pneumatic tank, to the loading part; and a sliding part disposed at one side in the horizontal portion and configured to ligate the titanium or polymer clip depending on the turning operation of the trigger part, the loading part may include: a casing part having a void therein and formed in a cylindrical shape; a rod part configured to move forward or rearward in the horizontal direction in the casing part; a fastening part recessed in a lower portion of the casing part so as to correspond to the stopper part and inserted into and fastened to the stopper part; and an elastic part configured to elastically press the rod part so that the rod part is in contact with one side of the flow path part, and the sliding part may include: a base part having one side being in contact with the shaft unit, and the other side extending in the horizontal direction so as to be coupled to the loading part; a movable part installed in the base part so as to reciprocatingly and rectilinearly move in the horizontal direction in conjunction with the rod part; and a protrusion portion protruding from a lower portion of the base part so as to correspond to the concave groove portion and configured to move the base part forward or rearward depending on the turning direction of the trigger part.

According to the surgical clip applier according to the present disclosure, the handle unit operates only once to automatically load and move the titanium or polymer clip to the application position by means of the pneumatic pressure, and the ligation unit ligates the titanium or polymer clip in the loaded state. Therefore, it is possible to maximize reliability and satisfaction by improving stability and accuracy in applying the clip regardless of the types of clips.

In addition, according to the surgical clip applier according to the present disclosure, the clip being stored in the cartridge unit or the shaft unit is automatically loaded to the application position on the ligation unit by the pneumatic pressure. Therefore, it is possible to achieve the convenience for practitioners, i.e., users, by improving stability and accuracy in applying the clip regardless of the skill of the practitioner. Therefore, it is possible to achieve the patient's quick recovery by shortening operating time and maximize the patient's satisfaction by preventing a medical accident.

Moreover, according to the surgical clip applier according to the present disclosure, the handle unit may be used in common for the titanium and polymer clips regardless of the types of clips, and only the shaft unit detachably coupled and installed to one side of the handle unit is replaced so as to be selectively replaceable depending on the types of clips including the titanium and polymer clips to be applied by the shaft unit and the ligation unit. Further, when all the clips are consumed or the pneumatic pressure is exhausted, only the cartridge unit, the clip, or the pneumatic tank may be conveniently and quickly replaced, such that the handle unit may be repeatedly used. Therefore, it is possible to reduce the surgery costs and prevent the waste of resources.

Further, the respective components of the surgical clip applier according to the present disclosure are modularized, individually produced, and formed to be easily attached, detached, and assembled, and as a result, it is possible to reduce manufacturing costs and manufacturing time by improving assembly properties and productivity. Further, it is possible to achieve convenience of storage and transport by making the surgical clip applier compact and to maximize the consumer's satisfaction, expand medical benefits, reduce medical insurance premiums, improve national stability, and promote exports by reducing manufacturing costs of the surgical clip applier.

### [Description of the Drawings]

FIG. 1 is a perspective view illustrating a surgical clip applier according to an embodiment of the present disclosure in a state in which a titanium clip shaft unit and a cartridge unit are mounted.
FIG. 2 is a cross-sectional view taken along line A-A in FIG. 1.
FIG. 3 is a perspective view illustrating the surgical clip applier according to the embodiment of the present disclosure in a state in which a polymer clip shaft unit is mounted.
FIG. 4 is a cross-sectional view taken along line B-B in FIG. 3.
FIG. 5 is a perspective view illustrating a handle unit in a state in which the shaft unit is removed from the surgical clip applier according to the embodiment of the present disclosure.
FIG. 6 is a cross-sectional view taken along line C-C in FIG. 5.
FIG. 7 is a cross-sectional view taken along line C-C in FIG. 5 and illustrating a state in which a pneumatic pressure supply part of the handle unit illustrated in FIG. 5 is fully opened.
FIGS. 8 to 10 are conceptual views for explaining an operational principle in which a surgical clip is loaded and ligated by an operation of the handle unit of the surgical clip applier according to the embodiment of the present disclosure.

### [Detailed Description of Certain Inventive Embodiment]

Hereinafter, an apparatus for operating a main shaft of a machine tool according to an exemplary embodiment of the present disclosure will be described in detail with respect to the drawings. The following exemplary embodiments are provided as examples for fully transferring the spirit of the present disclosure to those skilled in the art. Therefore, the present disclosure is not limited to the exemplary embodiments described below and may be specified as other aspects. Further, in the drawings, a size and a thickness of the apparatus may be exaggerated for convenience. Like reference numerals indicate like constituent elements throughout the specification.

Advantages and features of the present disclosure and methods of achieving the advantages and features will be clear with reference to exemplary embodiments described in detail below together with the accompanying drawings. However, the present disclosure is not limited to the exemplary embodiments disclosed herein but will be implemented in various forms. The exemplary embodiments of the present disclosure are provided so that the present disclosure is completely disclosed, and a person with ordinary skill in the art can fully understand the scope of the present disclosure. The present disclosure will be defined only by the scope of the appended claims. Like reference numerals indicate like constituent elements throughout the specification. In the drawings, sizes and relative sizes of layers and regions may be exaggerated for clarity of description.

The terms used in the present specification are for explaining the exemplary embodiments, not for limiting the present disclosure. Unless particularly stated otherwise in the present specification, a singular form also includes a plural form. The terms such as "comprise (include)" and/or "comprising (including)" used in the specification do not exclude presence or addition of one or more other constituent elements, steps, operations, and/or elements, in addition to the mentioned constituent elements, steps, operations, and/or elements.

Hereinafter, a surgical clip applier according to an embodiment of the present disclosure will be described in detail with reference to the drawings. The following exemplary embodiments are provided as examples for fully transferring the spirit of the present disclosure to those skilled in the art. Therefore, the present disclosure is not limited to the exemplary embodiments described below and may be specified as other aspects. Further, in the drawings, a size and a thickness of the apparatus may be exaggerated for convenience. Like reference numerals indicate like constituent elements throughout the specification.

Advantages and features of the present disclosure and methods of achieving the advantages and features will be clear with reference to exemplary embodiments described in detail below together with the accompanying drawings. However, the present disclosure is not limited to the exemplary embodiments disclosed herein but will be implemented in various forms. The exemplary embodiments of the present disclosure are provided so that the present disclosure is completely disclosed, and a person with ordinary skill in the art can fully understand the scope of the present disclosure. The present disclosure will be defined only by the scope of the appended claims. Like reference numerals indicate like constituent elements throughout the specification. In the drawings, sizes and relative sizes of layers and regions may be exaggerated for clarity of description.

The terms used in the present specification are for explaining the exemplary embodiments, not for limiting the present disclosure. Unless particularly stated otherwise in the present specification, a singular form also includes a plural form. The terms such as "comprise (include)" and/or "comprising (including)" used in the specification do not exclude presence or addition of one or more other constituent elements, steps, operations, and/or elements, in addition to the mentioned constituent elements, steps, operations, and/or elements.

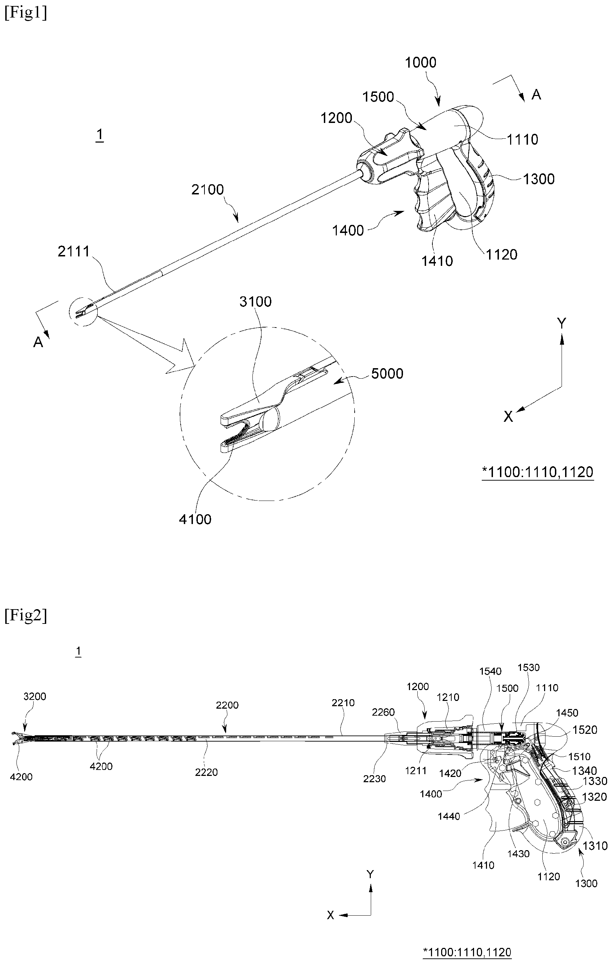

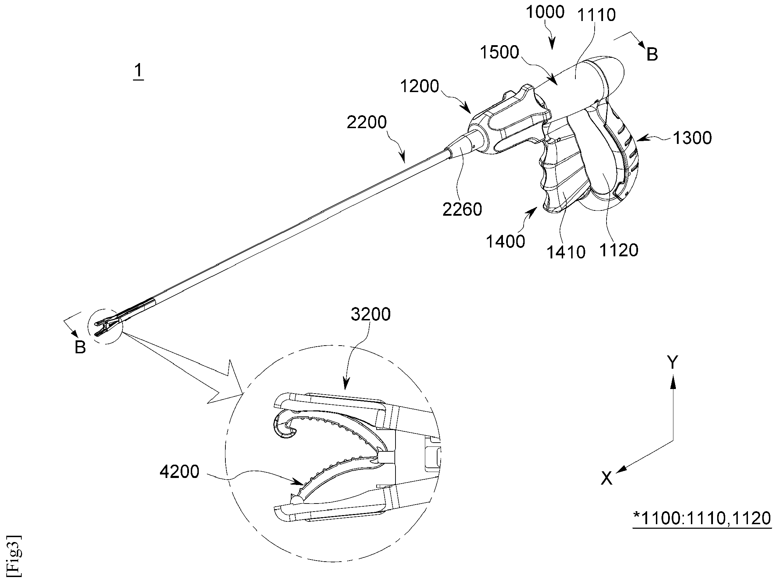

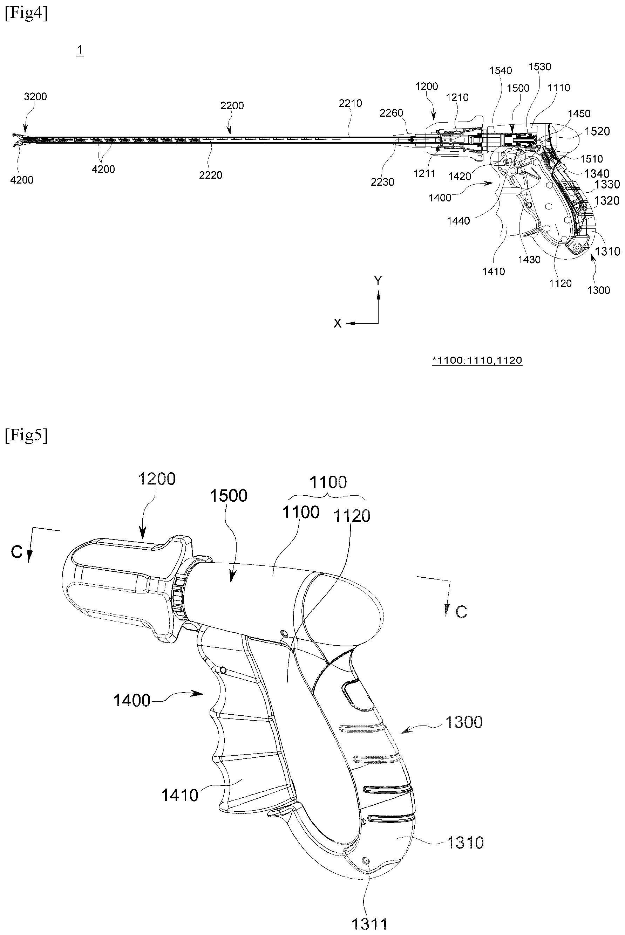

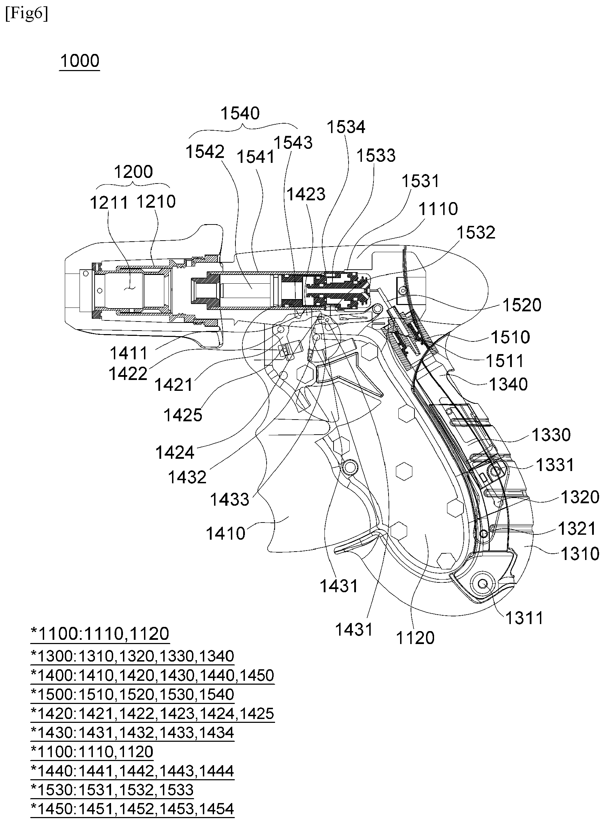

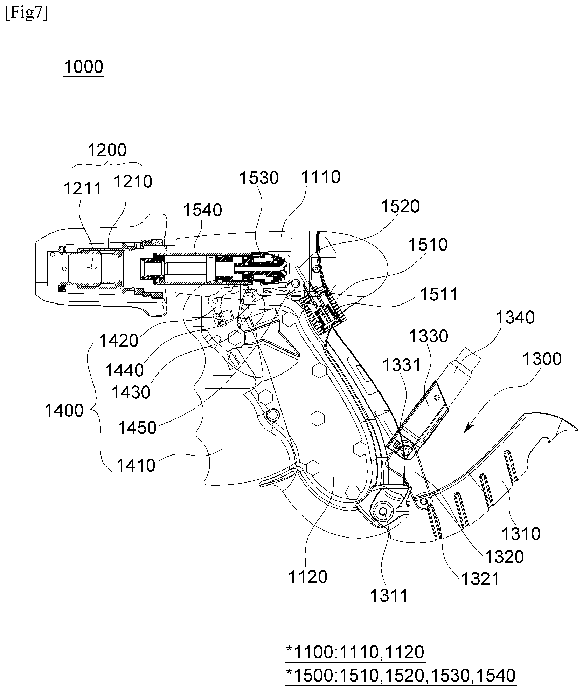

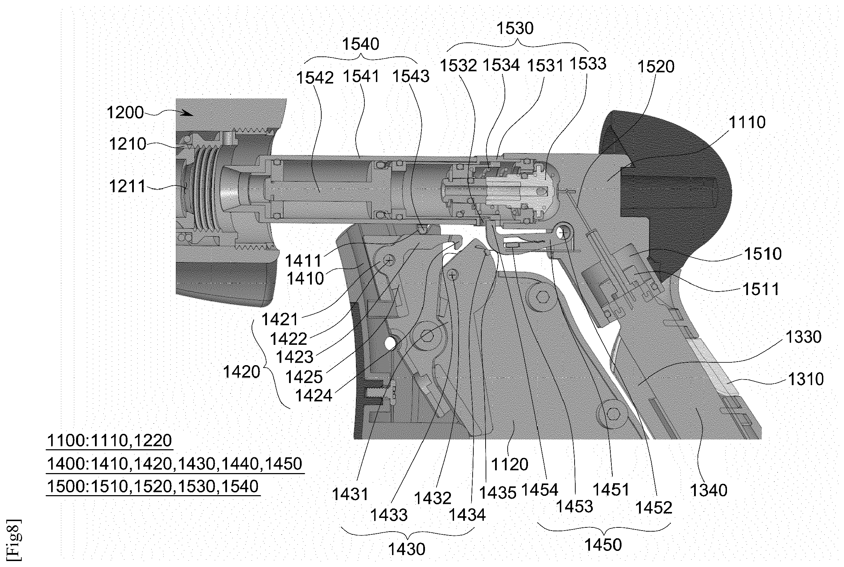

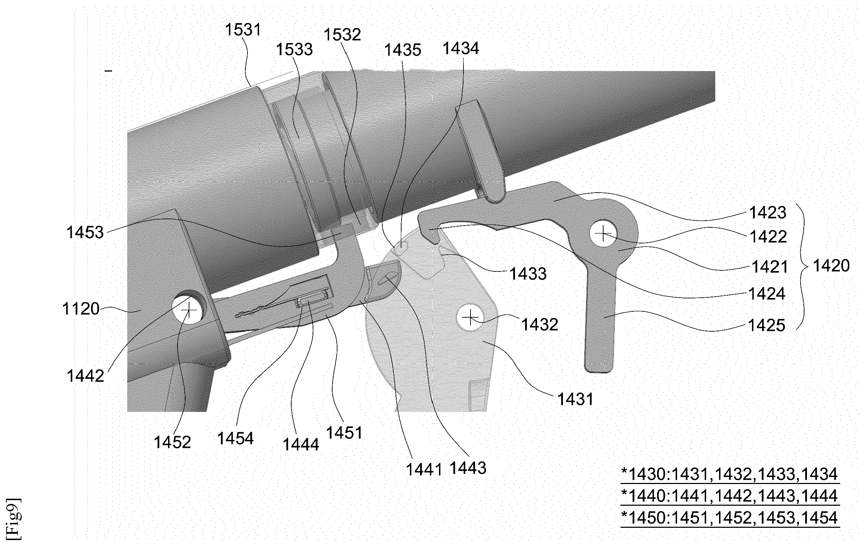

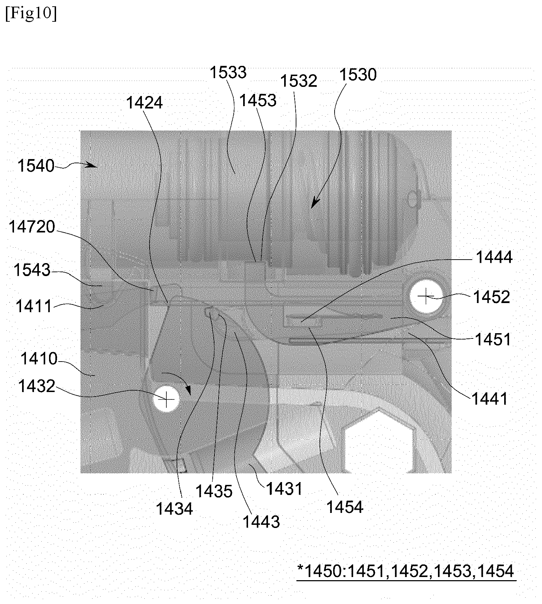

FIG. 1 is a perspective view illustrating a surgical clip applier according to an embodiment of the present disclosure in a state in which a titanium clip shaft unit and a cartridge unit are mounted, and FIG. 2 is a cross-sectional view taken along line A-A in FIG. 1. FIG. 3 is a perspective view illustrating the surgical clip applier according to the embodiment of the present disclosure in a state in which a polymer clip shaft unit is mounted, and FIG. 4 is a cross-sectional view taken along line B-B in FIG. 3. FIG. 5 is a perspective view illustrating a handle unit in a state in which the shaft unit is removed from the surgical clip applier according to the embodiment of the present disclosure, FIG. 6 is a cross-sectional view taken along line C-C in FIG. 5, and FIG. 7 is a cross-sectional view taken along line C-C in FIG. 5 and illustrating a state in which a pneumatic pressure supply part of the handle unit illustrated in FIG. 5 is fully opened. FIGS. 8 to 10 are conceptual views for explaining an operational principle in which a surgical clip is loaded and ligated by an operation of the handle unit of the surgical clip applier according to the embodiment of the present disclosure.

The terms used below are defined as follows. The "one side" means a left side of each constituent member illustrated in FIGS. 1 to 10, and the term "the other side" means a side opposite to one side of the same member, that is, a right side of each of the members illustrated in FIGS. 1 to 10. In addition, the term "one end" means an upper side of each of the constituent members illustrated in FIGS. 1 to 10, and the term "the other end" means a side opposite to one end of the same member, that is, a lower side of each of the members illustrated in FIGS. 1 to 10. If a handle unit is positioned at the left side unlike the configuration illustrated in FIGS. 1 to 10, one side and the other side are defined in an opposite manner. The term "horizontal direction" means a horizontal direction on the same member, that is, a direction from the right side to the left side or from the left side to the right side in FIGS. 1 to 10, and the term "vertical direction" means a vertical direction on the same member which is perpendicular to the horizontal direction, that is, a height direction from the lower side to the upper side or from the upper side to the lower side in FIGS. 1 to 10. In addition, the term "upward (upper)" means a "vertically upward direction", that is, a direction toward the upper side in FIGS. 1 to 10, and the term "downward (lower)" means a "vertically downward direction", that is, a direction toward the lower side in FIGS. 1 to 10. In addition, the term "inside (inner portion)" means an inner portion included in an interface of the same member and relatively close to a center of the same member, that is, an inner side of each of the components illustrated in FIGS. 1 to 10, and the term "outside" means an outer side of the same member or each of the components relatively distant from the center of the same member.

A surgical clip applier 1 according to the present disclosure will be described with reference to FIGS. 1 to 10. As illustrated in FIGS. 1 to 10, the surgical clip applier 1 according to the present disclosure includes a handle unit 1000, a shaft unit 2000, and a ligation unit 3000.

The handle unit 1000 serves to load a titanium clip 4100 or a polymer clip 4200 using pneumatic pressure and ligate a blood vessel or tubular tissue using the titanium clip 4100 or the polymer clip 4200.

The shaft unit 2000 is selectively and detachably installed at one side of the handle unit depending on the titanium clip 4100 or the polymer clip 4200 loaded by the pneumatic pressure.

The ligation unit 3000 is disposed at one side of the shaft unit and ligates the clip. That is, the blood vessel or the tubular tissue is ligated by the titanium or polymer clip as a trigger part of a lever part of the handle unit operates only once. Specifically, the ligation unit 3000 includes two components such as a pair of jaws which is disposed to face each other and retracted inward or spread outward by being operated by a trigger part, such that the two components operate to apply the clip to ligate the blood vessel or the tubular tissue. The titanium or polymer clip is moved and loaded to the application position on the ligation unit by the pneumatic pressure of the pneumatic tank of the pneumatic pressure supply part of the handle unit.

In addition, according to the surgical clip applier according to the present disclosure, the handle unit is used in common for the clips, and only the shaft unit coupled and installed to one side of the handle unit is replaced depending on the types of clips applied by the ligation unit.

That is, when the clip, which is applied by the ligation unit 3000, i.e., the titanium clip ligation unit 3100, is the titanium clip 4100, the cartridge unit 5000 configured to store a plurality of titanium clips is detachably coupled and installed in an accommodation portion 2111 formed at one side of the shaft part 2110 of the titanium clip shaft unit 2100 disposed adjacent to the ligation unit, and the titanium clip shaft unit 2100 is replaceably mounted on the handle unit 1000. When the clip, which is applied by the ligation unit, i.e., the polymer clip ligation unit 3200, is the polymer clip 4200, a polymer clip shaft unit 2200, which is disposed in a horizontal direction of a transfer part 2220, is replaceably and detachably mounted on the handle unit 1000 so that the plurality of polymer clips 4200 may be sequentially applied in the standby part 2221 of the transfer part 2220 of the shaft unit.

That is, as illustrated in FIGS. 1 and 2, when the titanium clip 4100 is used among the clips 4000 by the handle unit, the titanium clip shaft unit 2100 is selectively detachably installed at one side of the handle unit, such that the titanium clip 4100 is loaded to the titanium clip ligation unit 3100 disposed at one side of the titanium clip shaft unit 2100 and ligated to the blood vessel or the tubular tissue by the manipulation of the trigger part. Likewise, as illustrated in FIGS. 3 and 4, when the polymer clip 4200 is used among the clips 4000 by the handle unit, the polymer clip shaft unit 2200 is selectively detachably installed at one side of the handle unit, such that the polymer clip 4200 is loaded to the polymer clip ligation unit 3200 disposed at one side of the polymer clip shaft unit 2200 and ligated to the blood vessel or the tubular tissue by the manipulation of the trigger part.

Specifically, as illustrated in FIGS. 1 to 2, when the clip, which is applied by the ligation unit, i.e., the titanium clip ligation unit 3100, is the titanium clip 4100, the titanium clip is stored in the cartridge unit 5000. The cartridge unit 5000 for storing the plurality of titanium clips is replaceably and detachably installed in the accommodation portion 2111 of the shaft part 2110. The other side of the titanium clip shaft unit 2100 capable of storing and applying the titanium clip is replaceably inserted and installed at one side of the handle unit 1000 by penetrating a hollow portion 1211 of an adjustment part 1200 of the handle unit.

In addition, as illustrated in FIGS. 3 and 4, when the clip, which is applied by the ligation unit, i.e., the polymer clip ligation unit 3200, is the polymer clip 4200, the plurality of polymer clips 4200 is not stored in the cartridge unit, unlike the titanium clip, but disposed in a row in the horizontal direction of the polymer shaft unit in the polymer clip shaft unit 2200, i.e., the standby part 2221 of the transfer part 2220 so as to be sequentially applied. The other side of the polymer clip shaft unit 2200 capable of storing and applying the plurality of polymer clips 4200 is replaceably inserted and installed at one side of the handle unit 1000 by penetrating the hollow portion 1211 of the operating unit 1200 of the handle unit.

Therefore, according to the surgical clip applier according to the present disclosure, the handle unit may be used in common for the titanium and polymer clips regardless of the types of clips, and only the shaft unit detachably coupled and installed to one side of the handle unit is replaced so as to be selectively replaceable depending on the types of clips including the titanium and polymer clips to be applied by the shaft unit and the ligation unit. Further, when all the clips are consumed or the pneumatic pressure is exhausted, only the cartridge unit, the clip, or the pneumatic tank may be conveniently and quickly replaced, such that the handle unit may be repeatedly used. Therefore, it is possible to reduce the surgery costs and prevent the waste of resources.

In addition, as illustrated in FIGS. 1 to 7, the handle unit 1000 of the surgical clip applier 1 according to the embodiment of the present disclosure includes a housing part 1100, the adjustment part 1200, a pneumatic pressure supply part 1300, a lever part 1400, and an operation part 1500.

The housing part 1100 includes a horizontal portion 1110 extending in the horizontal direction, and a grip part 1120 disposed below the horizontal portion and extending in the vertical direction. That is, the housing part 1100 defines an external shape of the surgical clip applier. The housing part 1100 is provided in the form of a gun as a whole, but the present disclosure is not necessarily limited thereto. The handle unit 1000 may maintain the rigidity of the lever part, the operation part, the adjustment part, and the pneumatic pressure supply part. The horizontal portion and the grip part may be integrated to reduce manufacturing costs and manufacturing time. In addition, the housing part may be made of metal or plastic. The housing part serves as a handle so that a surgeon or a practitioner, who is a user, may hold and manipulate the surgical clip applier during a surgical procedure.

The adjustment part 1200 is detachably coupled and installed at one side of the horizontal portion, and a part of the shaft unit is inserted into the adjustment part 1200. That is, the practitioner, who is a user, may rotate the operation part clockwise or counterclockwise. Therefore, the shaft unit thus rotates clockwise or counterclockwise in a direction identical to the rotation direction of the operation part in conjunction with the operation part. The ligation unit provided at one side of the shaft unit thus rotates clockwise or counterclockwise in the direction identical to the rotation direction of the operation part in conjunction with the shaft unit, such that the user may easily manipulate and change the application position of the clip.

The adjustment part 1200 includes a coupling part 1210 disposed at a tip of the grip part, and the hollow portion 1211 formed by penetrating the inside of the coupling part. The titanium clip shaft unit or the polymer clip shaft unit is detachably coupled to the handle unit by penetrating the hollow portion.

The pneumatic pressure supply part 1300 is tiltably installed on the grip part and supplies the pneumatic pressure for loading the titanium or polymer clip. That is, as illustrated in FIGS. 1 to 7, the pneumatic pressure supply part 1300 includes a cover part 1310, a turning part 1320, a storage part 1330, and a pneumatic tank 1340.

The cover part 1310 is coupled to the grip part so as to be tiltable with respect to a tilting shaft 1311 disposed at the other end of the grip part.

The turning part 1320 is coupled to the cover part 1310 so as to be turnable with respect to a turning shaft 1321 disposed on a part of the cover part and turns in conjunction with the cover part.

The storage part 1330 is coupled to the turning part 1320 so as to be rotatable with respect to a rotary shaft 1331 disposed at one end of the turning part and rotates in conjunction with the turning part.

The pneumatic tank 1340 is separably inserted into the storage part 1330.

As described above, the turning part and the storage part are turned with respect to the turning shaft and the rotary shaft, respectively, in conjunction with the tilting operation of the cover part by the tilting operation of the cover part. Therefore, since the storage part is turned, the pneumatic tank may be easily replaced, the time required to replace the pneumatic tank may be shortened, the surgical operation time may be reduced, the convenience for the practitioner may be improved, the fatigue degree may be reduced, the recovery of the patient may be smoothly performed, and the satisfaction of the patient and the practitioner may be maximized.

In addition, the titanium or polymer clip is moved and loaded to the ligation position on the ligation unit by the pneumatic pressure supplied from the pneumatic tank by the manipulation of the trigger part. That is, the pneumatic tank 1340 is stored in the storage part of the pneumatic pressure supply part of the handle unit and stores the pneumatic pressure, i.e., power capable of automatically moving the clip to the application position on the ligation unit in conjunction with the manipulation of the trigger part. The pneumatic tank is filled with CO₂ gas, and a capacity of the pneumatic tank filled with the pneumatic gas may be variously set depending on the number of times the clip is loaded, but the present disclosure is not necessarily limited thereto. That is, capacities of the pneumatic tank charged with CO₂, may be set such that the clips are loaded 5 times, 10 times, 12 times, 15 times, or 20 times. In addition, when the overall pneumatic pressure in the pneumatic tank is used, the surgical clip applier may be used after conveniently replacing only the pneumatic tank. Further, the recovered pneumatic tank may be recharged with CO₂ gas, sterilized, and then reused. As a result, it is possible to prevent waste of resources, reduce manufacturing costs and maintenance costs, achieve miniaturization, allow a surgical procedure to be quickly performed to shorten a patient's recovery time, and maximize a practitioner's convenience.

The lever part 1400 includes the trigger part 1410 turnably installed on the grip part. The turning operation of the trigger part loads the titanium or polymer clip and ligates the titanium or polymer clip by operating the ligation unit.

The operation part 1500 is installed in the horizontal portion and operates in conjunction with the lever part. As illustrated in FIGS. 5 to 10, according to the surgical clip applier according to the present disclosure, the practitioner, who is the user, turns the trigger part of the lever part only once, such that the lever part and the operation part operate in conjunction with each other. Therefore, the clip is loaded to the application position on the ligation unit by the pneumatic pressure and ligated to the blood vessel or the tubular tissue after the clip is loaded.

According to the surgical clip applier according to the present disclosure, the handle unit operates only once to automatically load and move the titanium or polymer clip to the application position by means of the pneumatic pressure, and the ligation unit ligates the titanium or polymer clip in the loaded state. Therefore, it is possible to maximize reliability and satisfaction by improving stability and accuracy in applying the clip regardless of the types of clips.

As illustrated in FIGS. 5 to 10, according to the surgical clip applier 1 according to the present disclosure, the lever part 1400 of the handle unit 1000 includes the trigger part 1410, a drive part 1420, an actuator part 1430, a transmission part 1440, and a fixing part 1450.

The trigger part 1410 extends vertically downward from one side of the grip part 1120 of the housing part and is turnably coupled to a part of the grip part. The trigger part is turned when the practitioner pulls the trigger part. In addition, the trigger part 1410 includes a concave groove portion 1441 recessed at a vertical upper end of the trigger part. That is, when the trigger part 1441 is turned by being pulled by the practitioner, a protrusion portion 1543 of a sliding part inserted into the concave groove portion operates in conjunction with the trigger part 1441, such that the sliding part moves forward, and the inner tube 2120 of the titanium shaft unit or the round bar part 2210 of the polymer shaft unit moves forward and operates the titanium clip ligation unit or the polymer clip ligation unit to ligate the clip.

The drive part 1420 is installed in the grip part and disposed adjacent to the trigger part so as to be turned in a direction opposite to the turning direction of the trigger part 1410. As illustrated in FIGS. 5 to 10, the drive part 1420 includes a main body part 1421, a first leg part 1423, a second leg part 1425, and a ring part 1424. The drive part has a ' 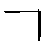 ' shape as a whole.

The driving shaft 1422 is disposed at a center of the main body part 1421, and the main body part 1421 is turned in the direction opposite to the turning direction of the trigger part 1410 with respect to the driving shaft.

The first leg part 1423 extends in the horizontal direction from an upper portion of the main body part.

The second leg part 1425 extends in the vertical direction from a lower portion of the main body part.

The ring part 1424 extends vertically downward from a tip of the first leg part and is selectively fixed and coupled to a catching projection part 1433 of the actuator part to be described below.

The actuator part 1430 is installed in the grip part and disposed adjacent to the drive part so as to be turned in the direction opposite to the turning direction of the drive part 1420. As illustrated in FIGS. 5 to 10, the actuator part 1430 includes a body part 1431, a catching projection part 1433, and a guide part 1434.

A rotation shaft 1432 is disposed on a part of the body part 1431, and the body part 1431 is turned with respect to the rotation shaft in the direction opposite to the turning direction of the drive part.

The catching projection part 1433 is recessed vertically downward from an upper portion of the body part so as to be selectively fixed and coupled to the ring part 1424 in the turning direction of the actuator part.

The guide part 1434 protrudes adjacent to the transmission part so as to have a predetermined curvature at a vertical upper end of the body part. That is, the guide part 1434 has a guide surface having a predetermined curvature. The cam follower part 1443 slides along the guide surface, such that the transmission part moves upward or downward in the turning direction of the actuator part.

The transmission part 1440 is installed in the horizontal portion in a state in which the other side thereof is fixed and coupled to a part of the horizontal portion 1110 so that one side thereof moves upward or downward in the turning direction of the actuator part 1430. As illustrated in FIGS. 5 to 10, the transmission part 1440 includes a body part 1441, a cam follower part 1443, and a pressing part 1444.

The body part 1441 has one side formed as a free end, and the other side fixed to a part of the horizontal portion and extending in the horizontal direction. That is, the body part has the other side fixed to the horizontal portion, and one side formed as a free end. The body part moves upward or downward with respect to a coupling shaft 1442 by the sliding operations of the cam follower part and the guide part.

The cam follower part 1443 is formed at one side of the body part, has a curvature corresponding to a curvature of the guide part, and slides along the guide surface 1435 of the guide part.

The pressing part 1444 protrudes from a center of a lateral surface of the body part toward the fixing part. When one side of the body part moves downward, the pressing part 1444 presses downward a stepped portion of the fixing part, such that the fixing part moves downward. Therefore, the stopper part 1453 is released from a fastening part 1532, and the pneumatic pressure supplied from the pneumatic tank is supplied through a flow path part, such that a rod part 1533 moves forward.

The fixing part 1450 is installed in the horizontal portion and disposed adjacent to the transmission part in a state in which the other side thereof is fixed and coupled to a part of the horizontal portion 1110 so that one side thereof moves upward or downward in conjunction with the transmission part 1440. As illustrated in FIGS. 5 to 10, the fixing part 1450 includes a support part 1451, the stopper part 1453, and the stepped portion 1454.

The support part 1451 has one side formed as a free end, and the other side fixed to a part of the horizontal portion and extending in the horizontal direction. The support part 1451 is parallel to the body part and disposed adjacent to the body part. That is, the support part 1451 has the other side fixed to the horizontal portion, and one side formed as a free end and disposed adjacent to the body part. The support part 1451 is moved upward or downward with respect to a stationary shaft 1452 by the pressing part in conjunction with the upward or downward movement of the body part. When the support part moves downward, the stopper part separates from the fastening part, and the flow path part is opened, such that the rod part is moved forward by the pneumatic pressure supplied from the pneumatic tank. The forward movement of the rod part moves a push rod or drawbar forward and automatically loads the clip to the application position.

The stopper part 1452 extends from a tip at one side of the support part to a vertical upper side, is inserted into the fastening part, and inhibits the forward movement of the rod part.

The stepped portion 1454 is formed in a stepped manner on the opening portion 1455 that penetrates a center of the support part so as to correspond to the pressing part so that the support part moves upward or downward in conjunction with the upward or downward movement of the body part. The pressing part is in close contact with the stepped portion 1454.

As described above, the drive part is turned in the direction opposite to the turning direction of the trigger part in conjunction with the turning operation of the trigger part. The actuator part is turned in the direction opposite to the turning direction of the drive part in conjunction with the turning operation of the drive part. One side of the transmission part moves downward in conjunction with the turning operation of the actuator part, and the fixing part moves downward in conjunction with the downward movement of one side of the transmission part, such that the pneumatic pressure supplied from the pneumatic tank is transmitted to the operation part, such that the operation part moves the push rod of the titanium shaft unit forward or moves the drawbar of the polymer shaft unit forward, thereby loading the titanium clip stored in the cartridge unit or the polymer clip stored in the transfer part to the application position on the ligation unit.

As illustrated in FIGS. 5 to 10, the operation part 1500 of the handle unit 1000 of the surgical clip applier 1 according to the present disclosure includes a base part 1510, a flow path part 1520, a loading part 1530, and a sliding part 1540.

The base part 1510 has a joint part 1511 coupled to communicate with the pneumatic tank while reciprocatingly and rectilinearly moving in the base part 1510, and the base part 1510 is fixed and installed to the other side in the horizontal portion.

The flow path part 1520 has one side communicating with the loading part, and the other side communicating with the base part, such that the flow path part 1520 transmits the pneumatic pressure, discharged from the pneumatic tank, to the loading part. That is, a nozzle of the flow path part 1520 is opened by the loading part when the practitioner turns the trigger part, thereby transmitting the pneumatic pressure, discharged from the pneumatic tank, to the loading part through the flow path part. In addition, to easily replace the pneumatic tank, the pneumatic tank and the base part communicate with each other through a link and turning structure with the cover part, the turning part, and the storage part, and the flow path part is penetratively formed to be inclined vertically downward at the other side in the horizontal portion of the housing part so as to communicate with the base part. Therefore, it is possible to minimize a size of the horizontal portion of the housing part. Further, since the storage part and the turning part of the pneumatic pressure supply part are arranged at an optimal position, it is possible to make the housing part compact, maintain rigidity while reducing manufacturing costs, maintain rigidity while making the surgical clip applier compact, and improve stability, accuracy, and reliability.

The loading part 1530 is disposed at the other side in the horizontal portion so as to be adjacent to the base part and loads the titanium or polymer clip in conjunction with the upward or downward movement of the stopper part. As illustrated in FIGS. 5 to 10, the loading part 1530 includes a casing part 1531, a fastening part 1532, a rod part 1533, and an elastic part 1534.

The casing part 1531 has a void therein and is formed in a cylindrical shape. The casing part defines an external shape of the loading part.

The fastening part 1532 is recessed in a lower portion of the casing part so as to correspond to the stopper part, and the stopper part is inserted and fastened to the fastening part 1532.

The rod part 1533 is moved forward or rearward in the horizontal direction in the casing part by the pneumatic pressure supplied from the pneumatic tank through the flow path part.

The elastic part 1534 elastically presses the rod part so that the rod part is in contact with one side of the flow path part in the casing part. That is, the rod part 1533 is always disposed to be moved rearward by the elastic part 1534 in the casing part. When the stopper part is separated from the fastening part by the manipulation of the trigger part, the rod part is moved forward by the pneumatic pressure supplied from the pneumatic tank, and the rod part is moved rearward again to the original position by the elastic part when the pneumatic pressure is discharged to the outside through the ligation unit, such that the stopper part is automatically inserted into the fastening part. That is, the elastic part may constitute the simple and compact loading part.

The sliding part 1540 is moved forward or rearward in the horizontal portion at one side in the horizontal portion by the turning operation of the trigger part so as to ligate the titanium or polymer clip. As illustrated in FIGS. 5 to 10, the sliding part 1540 includes a base part 1541, a movable part 1542, and a protrusion portion 1543.

The base part 1541 extends in the horizontal direction so that one side thereof is in contact with the shaft unit, and the other side thereof is coupled to the loading part. That is, the base part defines an external shape of the sliding part and is formed in a cylindrical shape having a void therein.

The movable part 1542 is installed in the base part so as to reciprocatingly and rectilinearly move in the horizontal direction in conjunction with the rod part. That is, when the rod part moves forward, the movable part moves forward, and the push rod or draw bar being in contact with the movable part moves forward in conjunction with the movable part, thereby loading the clip to the application position of the ligation unit.

The protrusion portion 1543 protrudes from a lower portion of the base part so as to correspond to the concave groove portion, thereby moving the base part (sliding part) forward or rearward in the turning direction of the trigger part. That is, since the protrusion portion 1543 is inserted into and fastened to the concave groove portion, the protrusion portion is turned in the turning direction of the trigger part in conjunction with the trigger part, and the turning operation of the protrusion portion is converted into the rectilinear movement, such that the sliding part moves forward or rearward. The forward or rearward movement of the sliding part moves the base part forward or rearward. The inner tube or the round bar part is moved forward or rearward by the base part in conjunction with the forward or rearward movement of the base part, such that the ligation unit operates to ligate the clip.

Therefore, the respective components of the surgical clip applier according to the present disclosure are modularized, individually produced, and formed to be easily attached, detached, and assembled, and as a result, it is possible to reduce manufacturing costs and manufacturing time by improving assembly properties and productivity. Further, it is possible to achieve convenience of storage and transport by making the surgical clip applier compact and to maximize the consumer's satisfaction, expand medical benefits, reduce medical insurance premiums, improve national stability, and promote exports by reducing manufacturing costs of the surgical clip applier.

An operational principle in which the handle unit of the surgical clip applier 1 according to the present disclosure operates only once to automatically load and move the titanium or polymer clip to the application position by means of the pneumatic pressure, and the ligation unit ligates the titanium or polymer clip in the loaded state and an operational principle of mounting or replacing the pneumatic tank will be described with reference to FIGS. 5 to 10.

First, in order to automatically load the clip using the pneumatic pressure, a new pneumatic tank needs to be mounted in the storage part of the pneumatic pressure supply part or the pneumatic tank from which the pneumatic pressure is completely discharged needs to be replaced. An operational principle of opening the storage part of the pneumatic pressure supply part will be described with reference to FIGS. 5 to 7. In order to open the storage part of the pneumatic pressure supply part, the user holds the cover part and tilts the cover part clockwise in FIG. 6 in a state in which the cover part comes into contact with the grip part and the cover part is completely closed as illustrated in FIG. 6, such that the cover part is tilted clockwise with respect to the tilting shaft as illustrated in FIG. 7. As illustrated in FIG. 7, when the cover part is tilted clockwise with respect to the tilting shaft, the turning part is turned counterclockwise in conjunction with the cover part with respect to the turning shaft disposed on a part of the cover part. At the same time, the storage part is turned clockwise in conjunction with the turning part with respect to the rotary shaft disposed at one end of the turning part, such that the storage part is naturally exposed to the outside. In this case, the user may remove and replace the pneumatic tank already mounted in the storage part with a new pneumatic tank or mount a new pneumatic tank in the storage part which is empty without any component mounted therein. Thereafter, when the user holds the cover part and tilts the cover part counterclockwise, the operation is performed in a reverse way to the above-mentioned operation. Therefore, the storage part is seated in the grip part, the cover part comes into contact with the grip part, and the pneumatic pressure supply part is closed.

On the premise that the pneumatic tank is mounted in the storage part, the operational principle of automatically loading and ligating the clip will be described with reference to FIGS. 8 to 10. As illustrated in FIGS. 8 to 10, the pneumatic tank is coupled to the joint part of the base part in the state in which the pneumatic tank is mounted in the storage part, such that the pneumatic pressure of the pneumatic tank is transmitted to the flow path part. However, in this case, when the rod part of the loading part is elastically pressed by the elastic part toward the other side of the horizontal portion, one side of the flow path part is closed to cut off the supply of the pneumatic pressure, and the stopper part of the fixing part is inserted into and fastened to the fastening part of the casing part to basically maintain a state in which the forward movement of the rod part by the pneumatic pressure is inhibited.

Thereafter, when the user turns the trigger part in one direction, the main body part of the drive part is turned with respect to the driving shaft in the direction opposite to the turning direction of the trigger part, the first leg part and the ring part disposed at the tip of the first leg part are also turned in the direction identical to the turning direction of the main body part in conjunction with the main body part. At the same time, the body part of the actuator part is turned with respect to the rotation shaft in the direction opposite to the turning direction of the main body part, and the catching projection part disposed at the upper end of the body part is also turned in the direction identical to the turning direction of the body part. That is, the ring part and the catching projection part are turned in the opposite directions so as to become close to each other, and the ring part is inserted into and fastened to the catching projection part. At the same time, the body part rotates about the rotation shaft in the direction opposite to the direction of the main body part, such that the guide part disposed on the upper portion of the body part is also turned in the same direction as the body part. As the guide part is turned, the cam follower part formed at one side of the body part of the transmission part moves downward along the guide surface of the guide part, and the pressing part moves downward in conjunction with the downward movement of the cam follower part. As the pressing part moves downward, the stepped part of the fixing part being in contact with the pressing part moves downward, the support part moves downward in conjunction with the stepped part as the stepped part moves downward, and the stopper part moves downward and separates from the fastening part in conjunction with the downward movement of the support part, such that the pneumatic pressure supplied from the pneumatic tank moves the rod part forward along the flow path part. As the rod part moves forward, the movable part moves forward in conjunction with the rod part, and the push rod or the draw bar moves forward in conjunction with the forward movement of the movable part and automatically loads the clip to the application position. Thereafter, the push rod and the drawbar are moved rearward by elastic force of a second coil spring or a second elastic member installed on the push rod and the drawbar and positioned at the original positions depend on the operational principle opposite to the above-mentioned principle.

In addition, as illustrated in FIGS. 8 to 10, when the trigger part is turned, the protrusion portion inserted into and fastened to the concave groove portion of the trigger part is primarily turned, the sliding part moves the base part forward, and the sliding part moves the inner tube or the round bar part forward, such that the ligation unit is turned to ligate the clip. Thereafter, as the trigger part is turned to the original position by the elastic member, the above-mentioned automatic loading operation is performed depending on the operational principle.

According to the surgical clip applier according to the present disclosure, the handle unit operates only once to automatically load and move the titanium or polymer clip to the application position by means of the pneumatic pressure, and the ligation unit ligates the titanium or polymer clip in the loaded state. Therefore, it is possible to maximize reliability and satisfaction by improving stability and accuracy in applying the clip regardless of the types of clips.

The handle unit 1000 of the surgical clip applier according to the present disclosure may exhibit an effect of preventing contamination by forming a coating layer using a coating composition excellent in the effect of preventing contamination.

More specifically, the coating composition includes inorganic nanoparticles, a silane compound, a surfactant, a binder, a dispersant, and a solvent.

An average diameter of the inorganic nanoparticle is 3 to 5 µm. When inorganic powders having small average diameters are within the above-mentioned range, the tactility will not deteriorate even if the coating layer is formed.

The inorganic nanoparticle is selected from a group consisting of barium titanate, silica (SiO₂), titanate (TiO₂), and a mixture thereof. Particularly, the inorganic nanoparticle is, but not limited to, barium titanate.

In particular, the silane compound is a compound expressed by the following Chemical Formula 1.

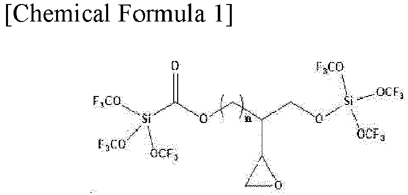

Here, n is an integer between 1 and 100.

The silane compound contains a number of -OCF3 substituents, and a fluorine film is formed in the coating layer by the -OCF3 substituents, thereby improving an anti-fouling effect.

In addition, an alkylene group with the carbon numbers of 1 to 100 is contained as a linker, and the -OCF3 substituents may be disposed at two opposite sides of an ester group at a predetermined distance. Therefore, the problem of the interference with the inorganic nanoparticles in the coating layer may be prevented, thereby further improving the anti-fouling effect.

In particular, the linker is the alkylene group with the carbon numbers of 5 to 10, that is, n is an integer between 5 and 10. In a case in which the coating layer is formed within the above-mentioned range, it is possible to form a fluorine film capable of maximally improving the anti-fouling effect within a range of thickness of the coating layer. If n is less than 5, the anti-fouling effect may deteriorate due to interference between the inorganic nanoparticles and the - OCF3 substituents. If n is more than 10, a distance between the inorganic nanoparticles and the fluorine film in the coating layer increases, which may prevent the interference but degrade the anti-fouling effect implemented by the combination of the fluorine film and the inorganic nanoparticles.

The surfactant may be a betaine-based surfactant, and more specifically, the surfactant may be selected from a group consisting of alkyl betaine-based, amide betaine-based, sulfobetaine-based, hydroxysulfobetaine-based, amidosulfobetaine-based, phosphobetaine-based, and imidazoliniumbetaine-based surfactants, but the present disclosure is not limited to this example.

The binder may be selected from a group consisting of an acrylic-based binder, a urethane-based binder, a silicone-based binder, and a mixture thereof. Specifically, the binder is a silicone-based binder. More specifically, the binder may be selected from a group consisting of polydimethylsiloxane, polyhydrosiloxane, and a mixture thereof, but the present disclosure is not limited to this example.

The dispersant may be made of a well-known component employed in the art as a dispersant for a carbon-based filler. More specifically, the dispersant may be selected from a group consisting of a polyester-based dispersant, a polyphenylene ether-based dispersant, a polyolefin-based dispersant, an acrylonitrile-butadiene-styrene copolymer dispersant, a polyarylate-based dispersant, a polyamide-based dispersant, a polyamide imide-based dispersant, a polyarylsulfone-based dispersant, a polyether imide-based dispersant, a polyether sulfone-based dispersant, a polyphenylene sulfide-based dispersant, a polyimide-based dispersant, a polyether ketone-based dispersant, a polybenzoxazole-based dispersant, a polyoxadiazole-based dispersant, a polybenzothiazole-based dispersant, a polybenzimidazole-based dispersant, a polypyridine-based dispersant, a polytrizaole-based dispersant, polypyrrolidine-based dispersant, a polydibenzofuran-based dispersant, a polysulfone-based dispersant, a polyurea-based dispersant, a polyurethane-based dispersant, polyphosphazene-based dispersant, and a mixture thereof, but the present disclosure is not limited to this example.

The solvent may be selected from a group consisting of water, alcoholic solvent, halogen-containing hydrocarbonic solvent, ketonic solvent, cellosolve solvent, and amidic solvent, but the present disclosure is not limited to this example.

The coating composition may include the inorganic nanoparticles, the silane compound, the surfactant, the binder, the dispersant, and the solvent. Particularly, based on 100 parts by weight of the solvent, the coating composition may include 5 to 20 parts by weight of the inorganic nanoparticles, 20 to 50 parts by weight of the silane compound, 10 to 20 parts by weight of the surfactant, 30 to 50 parts by weight of the binder, and 5 to 10 parts by weight of the dispersant. Within the above-mentioned range, a synergy effect implemented by interactions between extracts exhibits a synergy effect with critical significance. However, the synergy effect rapidly deteriorates or almost disappears out of the above-mentioned range.

The coating composition is manufactured by mixing the inorganic nanoparticles, the silane compound, the surfactant, the binder, the dispersant, and the solvent and then stirring the resulting mixture at 100 to 150 rpm. The manufactured coating composition has viscosity of 1,500 to 2,000 cps. A predetermined shape of a substrate may be maintained when forming the coating layer on a surface of the substrate with the range of the viscosity. Therefore, the coating may be easily performed using a well-known coating method.

### [Preparation Example 1: Manufacture of coating composition]

The coating composition was manufactured by mixing the barium titanate with an average diameter of 3 to 5 µm and a silane compound, an alkylbetaine-based surfactant, a polyhydrosiloxane binder, water, and a polyester-based dispersant, which were expressed in the following Chemical Formula 1, and then stirring the resulting mixture for 10 to 30 minutes at 100 rpm.

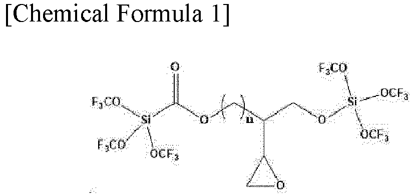

(Here, n is an integer of 10.)

All the components constituting the coating composition were purchased and used to manufacture the coating composition. The following Table 1 shows the more specific composition of the coating composition.

**[Table 1]**

| | BB1 | BB2 | BB3 | BB4 | BB5 | BB6 | BB7 | BB8 | BB9 | BB10 | BB11 |
|---|---|---|---|---|---|---|---|---|---|---|---|
| Water | 100 | 100 | 100 | 100 | 100 | 100 | 100 | 100 | 100 | 100 | 100 |
| Barium titanate | 1 | 5 | 10 | 15 | 20 | 25 | 15 | 15 | 15 | 15 | 15 |
| Silane compound | 10 | 20 | 30 | 40 | 50 | 60 | 40 | 40 | 40 | 40 | 40 |
| Surfactant | 5 | 10 | 13 | 16 | 20 | 30 | 16 | 16 | 16 | 16 | 16 |
| Binder | 20 | 30 | 35 | 45 | 50 | 60 | 45 | 45 | 45 | 45 | 45 |
| Dispersant | 1 | 5 | 7 | 8 | 10 | 20 | 8 | 8 | 8 | 8 | 8 |

The coating layer was manufactured by immersing a 5×5cm specimen in the coating composition of BB1 to BB11 and drying the specimen for 60 minutes at 90 to 95°C.

### [Experimental Example 1: Evaluation of external appearance]

The viscosity was measured using the Brookfield viscometer at 25°C before coating the specimen using the coating compositions of BB1 to BB11. The external appearance was observed after the coating layer was formed.

The evaluation was performed depending on the following evaluation criteria in accordance with the case in which the flat coating layer is formed on the surface of the substrate and the degree to which the coating layer is curved.
∘: Flat coating layer is formed
△: Coating layer with partial curved shape is formed
×: Coating layer curved in large area is formed

**[Table 2]**

| | | | | | | |
|---|---|---|---|---|---|---|
| | BB1 | BB2 | BB3 | BB4 | BB5 | BB6 |
| Viscosity (cps) | 500 | 1,120 | 1,560 | 1,800 | 1,920 | 2,250 |
| Evaluation of external appearance | X | X | ○ | ○ | ○ | △ |

As shown in Table 2, it can be ascertained that the result of forming the coating layer on the substrate and evaluating the external appearance indicates that the viscosity varies depending on the content range of the coating composition and the viscosity greatly varies depending on whether the uniform coating layer is formed in accordance with the viscosity range.

### [Experimental Example 2: Evaluation of contamination resistance]

The specimen having the surface coated with experiment numbers BB1 to BB6 was placed on a rubbing fastness tester (model name: DL-2007), a test fabric (product name: IEC carbon black/mineral oil, from EMPA) was positioned on a surface of a contaminated sample, friction was applied reciprocatingly ten times, the contaminated samples were compared with the naked eye, and contamination ratings were assigned as shown in the following Table 2.

The evaluation criteria are as follows.
⊚: Contamination is not completely visible
∘: Small amount of contamination is visible or contamination is almost invisible
△: Contamination is slightly severe
X: Contamination is significantly severe

**[Table 3]**

| | | | | | | |
|---|---|---|---|---|---|---|
| | BB1 | BB2 | BB3 | BB4 | BB5 | BB6 |
| Contamina tion resistance | X | Δ | ⊚ | ⊚ | ○ | △ |

Referring to Table 3, it is ascertained that the contamination is significantly severe in the case of BB1. The contamination is slightly severe in the case of BB2 and BB6. However, it can be ascertained that no contamination is visible and contamination resistance is excellent in the case of BB3 to BB5.

While the present disclosure has been described above with reference to the exemplary embodiments of the present disclosure in the detailed description of the present disclosure, it may be understood, by those skilled in the art or those of ordinary skill in the art, that the present disclosure may be variously modified and changed without departing from the spirit and scope of the present disclosure disclosed in the claims. Accordingly, the technical scope of the present disclosure should not be limited to the contents disclosed in the detailed description of the specification but should be defined only by the claims.

While the present disclosure has been described above with reference to the exemplary embodiments of the present disclosure in the detailed description of the present disclosure, it may be understood, by those skilled in the art or those of ordinary skill in the art, that the present disclosure may be variously modified and changed without departing from the spirit and scope of the present disclosure disclosed in the claims. Accordingly, the technical scope of the present disclosure should not be limited to the contents disclosed in the detailed description of the specification but should be defined only by the claims.

### [Description of Main Reference Numerals of Drawings]

- 1:: Surgical clip applier
- 1000:: Handle unit
- 1100:: housing part
- 1200:: Adjustment part
- 1300:: Pneumatic pressure supply part
- 1400:: Lever part
- 1500:: Operation part
- 2000:: Shaft unit
- 2100:: Titanium clip shaft unit
- 2200:: Polymer clip shaft unit
- 3000:: Ligation unit
- 3100:: Titanium clip ligation unit
- 3200:: Polymer clip ligation unit
- 4000:: Clip
- 4100:: Titanium clip
- 4200:: Polymer clip
- 5000:: Cartridge unit

## Claims

1. A surgical clip applier, which applies a titanium or polymer clip for ligating a blood vessel or tubular tissue, the surgical clip applier comprising:
a handle unit configured to load the titanium or polymer clip by pneumatic pressure and ligate the blood vessel or tubular tissue with the titanium or polymer clip;
a shaft unit selectively and detachably installed at one side of the handle unit depending on the titanium or polymer clip loaded by the pneumatic pressure; and
a ligation unit disposed at one side of the shaft unit and configured to ligate the clip,
wherein the handle unit comprises:
a housing part having a horizontal portion extending in a horizontal direction and a grip part disposed below the horizontal portion and extending in a vertical direction;
an adjustment part detachably coupled and installed at one side of the horizontal portion and configured to allow a part of the shaft unit to be inserted thereinto;
a pneumatic pressure supply part tiltably installed on the grip part and configured to supply the pneumatic pressure for loading the titanium or polymer clip;
a lever part having a trigger part turnably installed on the grip part and configured to load and ligate the titanium or polymer clip depending on a turning operation of the trigger part; and
an operation part installed in the horizontal portion and configured to operate in conjunction with the lever part.

2. The surgical clip applier of claim 1, wherein the adjustment part comprises:
a coupling part disposed at a tip of the grip part; and
a hollow portion configured to penetrate the inside of the coupling part, and
wherein the pneumatic pressure supply part comprises:
a cover part coupled to the grip part so as to be tiltable with respect to a tilting shaft disposed at the other end of the grip part;
a turning part coupled to the cover part so as to be turnable with respect to a turning shaft disposed on a part of the cover part and configured to turn in conjunction with the cover part;
a storage part coupled to the turning part so as to be rotatable with respect to a rotary shaft disposed at one end of the turning part and configured to rotate in conjunction with the turning part; and
a pneumatic tank separably inserted into the storage part.

3. The surgical clip applier of claim 2, wherein the lever part comprises:
a drive part installed in the grip part and disposed adjacent to the trigger part so as to turn in a direction opposite to the turning direction of the trigger part;
an actuator part installed in the grip part and disposed adjacent to the drive part so as to be turned in a direction opposite to the turning direction of the drive part;
a transmission part installed in the horizontal portion in a state in which the other side thereof is fixed and coupled to a part of the horizontal portion so that one side thereof moves upward or downward depending on the turning direction of the actuator part; and
a fixing part installed in the horizontal portion and disposed adjacent to the transmission part in a state in which the other side thereof is fixed and coupled to a part of the horizontal portion so that one side thereof moves upward or downward in conjunction with the transmission part.

4. The surgical clip applier of claim 3, wherein the trigger part comprises a concave groove portion recessed at an upper end of the trigger part,
wherein the drive part comprises:
a main body part having a driving shaft disposed at a center thereof and configured to turn with respect to the driving shaft in the direction opposite to the turning direction of the trigger part;
a first leg part extending in the horizontal direction from an upper portion of the main body part;
a second leg part extending in the vertical direction from a lower portion of the main body part; and
a ring part extending vertically downward from a tip of the first leg part,
wherein the actuator part comprises:
a body part having a rotation shaft disposed on a part thereof and configured to turn with respect to the rotation shaft in the direction opposite to the turning direction of the drive part;
a catching projection part recessed vertically downward from an upper portion of the body part so as to be selectively fixed and coupled to the ring part in the turning direction of the actuator part; and
a guide part protruding adjacent to the transmission part so as to have a predetermined curvature at a vertical upper end of the body part,
wherein the transmission part comprises:
a body part having one side formed as a free end, and the other side fixed to a part of the horizontal portion and extending in the horizontal direction;
a cam follower part formed at one side of the body part so as to have a curvature corresponding to a curvature of the guide part and configured to slide along a guide surface of the guide part; and
a pressing part protruding from a center of a lateral surface of the body part toward the fixing part, and
wherein the fixing part comprises:
a support part having one side formed as a free end, the other side fixed to a part of the horizontal portion and extending in the horizontal direction, the support part being parallel to the body part and disposed adjacent to the body part;
a stopper part extending vertically upward from one side tip of the support part; and
a stepped part formed in a stepped manner by penetrating a center of the support part so as to correspond to the pressing part so that the support part moves upward or downward in conjunction with the upward or downward movement of the body part, the stepped part being in close contact with the pressing part.

5. The surgical clip applier of claim 4, wherein the operation part comprises:
a base part fixed and installed at the other side in the horizontal portion and having a joint part disposed in the base part and coupled to the pneumatic tank so as to communicate with the pneumatic tank by reciprocatingly and rectilinearly moving;
a loading part provided at the other side in the horizontal portion, disposed adjacent to the base part, and configured to load the titanium or polymer clip depending on the upward or downward movement of the stopper part;
a flow path part having one side communicating with the loading part, and the other side communicating with the base part, and configured to transmit the pneumatic pressure, discharged from the pneumatic tank, to the loading part; and
a sliding part disposed at one side in the horizontal portion and configured to ligate the titanium or polymer clip depending on the turning operation of the trigger part,
wherein the loading part comprises:
a casing part having a void therein and formed in a cylindrical shape;
a rod part configured to move forward or rearward in the horizontal direction in the casing part;
a fastening part recessed in a lower portion of the casing part so as to correspond to the stopper part and inserted into and fastened to the stopper part; and
an elastic part configured to elastically press the rod part so that the rod part is in contact with one side of the flow path part, and
wherein the sliding part comprises:
a base part having one side being in contact with the shaft unit, and the other side extending in the horizontal direction so as to be coupled to the loading part;
a movable part installed in the base part so as to reciprocatingly and rectilinearly move in the horizontal direction in conjunction with the rod part; and
a protrusion portion protruding from a lower portion of the base part so as to correspond to the concave groove portion and configured to move the base part forward or rearward depending on the turning direction of the trigger part.
